Europäisches Patentamt

European Patent Office

Office européen des brevets

(19)

(11) Publication number: **0 340 002 B1**

## EUROPEAN PATENT SPECIFICATION

(12)

(45) Date of publication of patent specification: **16.03.94**

(51) Int. Cl.5: **C12P 21/00**, G01N 33/577, A61K 39/395

(21) Application number: **89304212.7**

(22) Date of filing: **27.04.89**

(54) **A human-human hybridoma derived monoclonal antibody, and the use thereof.**

(30) Priority: **27.04.88 JP 102360/88**
**12.08.88 JP 201507/88**

(43) Date of publication of application:
**02.11.89 Bulletin 89/44**

(45) Publication of the grant of the patent:
**16.03.94 Bulletin 94/11**

(84) Designated Contracting States:
**BE CH DE FR GB IT LI NL SE**

(56) References cited:
**EP-A- 0 157 574**
**EP-A- 0 178 891**
**EP-A- 0 182 467**
**US-A- 4 668 629**

**PROCEEDINGS OF THE NATIONAL ACADEMY OF SCIENCES OF THE UNITED STATES OFAMERICA, vol.77, no. 9, September 1980 (Balitmore, USA) L.OLSSON et al. "Human-human hybridomas producing mono- clonal antibodies of pre- defined antigenicspecifi-city." pages 5429-5431**

(73) Proprietor: **Tsuji, Kimiyoshi**
**3050-139, Kawashima-cho**
**Asahi-ku**
**Yokohama-shi Kanagawa-ken(JP)**

(72) Inventor: **Tsuji, Kimiyoshi**
**3050-139, Kawashima-cho**
**Asahi-ku**
**Yokohama-shi Kanagawa-ken(JP)**

(74) Representative: **Bizley, Richard Edward et al**
**BOULT, WADE & TENNANT**
**27 Furnival Street**
**London EC4A 1PO (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel monoclonal antibodies and, inter alia, an immunological method utilizing the same. More particularly, the invention relates to monoclonal antibodies derived from human-human hybridomas capable of recognizing a differentiated antibody of human lymphocytes and/or exhibiting immunosuppression against human lymphocytes and free from any adverse side effects when a human body is administered therewith, immunosuppressive agents with the same as the effective ingredients, as well as diagnostic methods utilizing the same.

Along with the rapid progress in the biotechnology in recent years, monoclonal antibodies are highlighted and many intensive and extensive Investigations are being undertaken in a variety of fields in relation to monoclonal antibodies.

As is well known, every living body of animal is provided with a function of self-defence called immunity which is brought to exhibition against a foreign body such as bacteria, referred to as an antigen, intruding the living body so that the lymphocytes in the body produce an antibody having an activity of eliminating or holding down the foreign body out of or in the living body. Under such a situation, lymphocytes of a single clone characteristically produce a single kind of the antibody which is called a monoclonal antibody.

Active investigations are now under way for the application of monoclonal antibodies in a variety of fields including biochemical analysis such as affinity chromatography, clinical diagnosis and therapy in the medical science and so on. The application of monoclonal antibodies in the clinical therapy is exemplified by:

(1) the so-called missile therapeutic method of cancer in which a monoclonal antibody, of which the antigen is the specific glycoprotein contained in the surface layer of the cancer cells, is prepared and administrated to a patient of cancer as combined with an anticancer agent or an anticancer toxin capable of killing the cancer cells so that the monoclonal antibody selectively adheres to the surface alone of the cancer cells by the antigen-antibody reaction to have the cancer cells destroyed by the anticancer agent or toxin connected to the antibody;

(2) the use of a monoclonal antibody in place of conventional immunosuppressive agents administrated to a patient of organ transplantation with an object ot mitigate the problem of rejection against the transplanted organ when the major histocompatible antigens are not in consistency with each other while conventional immunosuppressive agents sometimes lead to death of the patient due to heavy side effects or infectious diseases; and

(3) the use of a monoclonal antibody by utilizing the features of high specificity, stability of quality and low costs inf manufacture in place of conventional antiserums in the so-called gamma-globulin method in which an antibody called an antiserum prepared from human blood is administrated to a patient suffering various kinds of immunodeficiency syndromes or serious infectious diseases.

Several methods are known and practiced in the prior art for the preparation of a monoclonal antibody including a method in which an antibody-producing cell and a myeloma cell are subjected to cell fusion and the thus obtained hybridoma is cultured, a method in which lymphocytes are subjected to transformation with a virus and the thus obtained antibody-producing cell strain is cultured, and so on.

Needless to say, it is essential in the preparation of a human-type monoclonal antibody to obtain a human antibody-producing cell sensitized by a certian antigen and having specificity to the same antigen while in vivo stimulation with an antigen is generally not permitted in human bodies with a few exceptional cases. This situation is the reason for the unestablished practical technology for the above mentioned possible application of monoclonal antibodies against various kinds of antigens in general. An attempt has also been made to obtain a monoclonal antibody by means of a permanent cell of human obtained by an immortalizing means of a human antibody-producing cell, such as transformation by the Epstein-Barr virus (EBV), or a human hybridoma obtained by the cell fusion with a human myeloma cell. Different from the system of mice, unfortunately, no transforming cells useful as a parent strain have yet been established and no hybridoma having a stable activity for antibody production has yet been obtained in the system of human. Accordingly, the monoclonal antibodies available at present are limited to those derived from mice. Such a monoclonal antibody of mouse-origin may of course be useful in certian applications, for example, in the biochemical analysis, e.g., affinity chromatography, and clinical diagnosis. When the object of application of such a monoclonal antibody is a therapeutic treatment of patients of certain diseases, however, the monoclonal antibody is recognized by the human body administrated therewith as a foreign protein possibly to cause an allergic reaction so that the application of such a monoclonal antibody to a therapeutic treatment of patients is necessarily under limitation. It is therefore eagerly desired to develop a technology which provides a means to conveniently prepare a human monoclonal antibody applicable to a thereapeutic purpose.

As to a monoclonal antibody capable of recognizing a differentiated antigen of human lymphocytes and useful in thereapeutic treatments, no antibody derived from a human-human hybridoma has yet been disclosed although several mouse-origin ones are known.

As to a monoclonal antibody used as an immunosuppressive agent, on the other hand, OKT3 is known and practically used, mainly, in the United States with a purpose of preventing the rejection reaction against transplanted tissues (see, for example, Japanese Patent Kokai 55-145617). This antibody, however, is very defective because it is derived from mouse and acts as a foreign protein in human body to cause serious side effects when it is administrated to a patient more than once (see Nikkei Biotech, July 14 issue, 1986, and February 23 issue, 1987). Reportedly, patients administrated therewith in clinical cases suffered chill, shaking chill and pyrexia after 30 minutes from administration and a half of the patients fell into dyspnea. What was worse was that recurrence of the rejection reaction was noted in 12 to 16 cases according to the report in Rinsho Men-eki (clinical Immunology), volume 17, No. 10, page 895 (1985).

The present invention accordingly has an object, in view of the above described situation relative to the monoclonal antibodies, to provide a novel monoclonal antibody dervied from a human-human hybridoma capable of exhibiting an activity of recognizing a differentiation antigen of human lymphocytes or immunosuppressive effect against human lymphocytes and useful as a side effect-free therapeutic medicament of human diseases.

The invention facilitates a novel immunosuppressive method by utilizing a monoclonal antibody derived from a human-human hybridoma and administrable to human without the danger of side effects.

A further object of the invention is to provide a novel diagnostic method by utilizing a monoclonal antibody derived from a human-human hybridoma and capable of recognizing a differentiation antigen of human lymphocytes.

The monoclonal antibody of the invention is derived from a human-human hybridoma which has been deposited under the deposit number FERM BP-2384 and is capable of recognizing a differentiation antigen of human lymphocytes or capable of exhibiting an immunosuppressive activity against human lymphocytes.

In another aspect the invention provides a method for detecting or determining a differentiation antigen in a biological sample which comprises contacting the biological sample with a monoclonal antibody derived from a human-human hybridoma which has been deposited under the deposit number FERM BP-2384, the antibody being capable of recognizing the differentiation antigen of human lymphocytes, so as to form a complex of the monoclonal antibody and any lymphocytes in the biological sample having said differentiation antigen and detecting or determining the complex.

The above mentioned human-human hybridoma can be obtained by subjecting a transformed human cell to a treatment to inhibit the cell proliferation and then subjecting the thus treated transformed human cell to cell fusion with a human antibody-producing cell.

The above mentioned diagnostic method is performed by bringing a biological sample under testing into contact with the above defined monoclonal antibody so as to form a complex between the monoclonal antibody and the lymphocyte in the sample having a differentiated antigen and detecting or determining the thus formed complex.

DETAILED DESCRIPTION OF THE PREFERRED EMBODIMENTS

The inventor has conducted extensive investigations to prepare a human-human hybridoma having a stable activity for producing a human monoclonal antibody and previously disclosed in Japanese Patent Kokai 63-17688 that such a human-human hybridoma can be obtained by subjecting a transformed human cell to a treatment to inhibit the cell proliferation and then subjecting the thus treated transformed human cell to cell fusion with a human antibody-producing cells.

Further continued investigations have led to a novel discovery that the above mentioned human-human hybridoma produces a human monoclonal antibody capable of recognizing a differentiation antigen of human lymphocytes or exhibiting an immunosuppressive effect against human lymphocytes, that the thus obtained human monoclonal antibody can be used as an immunosuppressive agent without the danger of any side effect and that the human monoclonal antibody can be used in the detection or determination of human lymphocytes exhibiting a differentiation antigen by forming a complex between the monoclonal antibody and the lymphocytes.

The first step in the preparation of a human-human hybridoma having a stable antibody-producing activity is a treatment of a transformed human cell to inhibit the cell profiferation. Usable transformed human cells as the parent cell include, for example, human B cells, human hepatocytes, human splenocytes, tonsillar cells, human lymph node cells and the like, of which human B cells as transformed are particularly preferable. The transformation can be performed by a conventional method such as

EP 0 340 002 B1

infection of the cell with a virus and a genetic recombination method.

The method for inhibiting the proliferation of these transformed human cells includes chemical treatment, irradiation with high-energy radiation and the like. Preferably, the treatment to inhibit the proliferation of transformed human cells is performed by a chemical treatment with a reagent capable of inhibiting synthesis of protein or RNA such as emetine, actinomycin D and the like either singly or as a combination of two kinds or more. The amount of the chemical reagent to be used should be appropriately selected depending on the type of the cells to be subjected to cell fusion. As a rough measure, the amount of the reagent should be sufficient to completely kill the transformed human cells within 7 to 10 days. The transformed human cells after the treatment to inhibit the cell proliferation can be heterozygous or homozygous but homozygous ones are preferred in respect of the efficiency in the subsequent screening.

The next step is cell fusion between the transformed human cells after the treatment to inhibit the cell proliferation in the above described manner and antibody-producing human cells to produce a human-human hybridoma. Examples of the above mentioned antibody-producing cell include, for example, human B cells, human plasma cells, human hepatocytes, human embryonal hepatocytes, human splenocytes, human lymph node cells, cultured human B cells and the like, of which human B cells and cultured human B cells are preferred. The antibody-producing human cells can be used as isolated from a tissue or blood of human body but it is optional to use the same after a transformation treatment. It is generally desirable here that the cell fusion is followed by a treatment with an anti-HLA antibody to exclude the non-fusion antibody-producing human cells. The cell fusion is performed by a known method such as the method utilizing polyethylene glycol, virus and the like and the electric cell fusion method. Preferably, the cell fusion is performed by using the proliferation-inhibited human cells of 1 to 30 times in number based on the antibody-producing human cells. Various types of culture media can be used for the purpose of cell fusion including, for example, RPMI 1640 culture medium, MEM culture medium and modified Dulbecco's culture medium as well as serum-added and serum-free culture media thereof.

The human-human hybridoma obtained in this manner is then subjected to inspection of the desired antibody-producing activity and cloning.

The antibody-producing activity can be checked by the assay for the antibody produced by the human-human hybridoma. This antibody assay is performed by testing the reactivity thereof against a sensitized antigen according to one of various known methods for the detection of antibodies in general such as ELISA method described in Meth. of Enzymol., volume 70, pages 419 to 439 (1980), plaque method, spot method, agglutination reaction method, Ouchterlony method, enzyme immunoassay (EIA), radioimmunoassay (RIA), cytotoxicity assay and the like (see Hybridoma Method and Monoclonal Antibody, published by R & D Planning Co., pages 30 to 53, 1982, and elsewhere). The cloning can be easily performed by repeating the known step of sub-culture or by undertaking a conventional limiting dilution method.

The human-human hybridoma produced in this manner is cultured usually in a suitably culture medium such as RPMI 1640 culture medium, MEM culture medium and modified Dulbecco's culture medium as well as serum-added and serum-free culture media thereof at a temperature of 36.5 to 37.5 °C with a pH of the medium controlled in the range of 6.7 to 7.5 or, preferably, 7.0 to 7.3 and a concentration of dissolved oxygen of 0.5 to 6 ppm or, preferably, 3 to 5 ppm. When the pH of the culture medium is outside the above mentioned range, a retarding effect is caused on the rate of hybridoma multiplication, especially, in the initial stage. When the concentration of the dissolved oxygen is outside the above mentioned rage, an adverse effect is caused on the productivity of the desired antibody by the hybridoma. The vessel used for culturing is not particularly limitative including microplates, Petri dishes, glass flasks, culture tanks and the like depending on the desired scale of culturing.

The human hybridoma cultured in this manner produces the desired human-type monoclonal antibody and secretes the same in the supernatant of the culture medium. The human monoclonal antibody can be isolated and purified from the culture medium by one or a combination of conventional methods including centrifugal separation, ultrafiltration, gel filtration, ion-exchange chromatography, affinity chromatography and the like. In particular, a good efficiency is obtained in the isolation and purification thereof by the method of affinity chromatography using a column loaded with protein A.

Being derived from a human-human hybridoma, the monoclonal antibody obtained in this manner can be used as an immunosuppressive agent for human without being accompanied by any side effect.

When the human-human hybridoma is produced by appropriately selecting the transformed human cell and the human antibody-producing cell used in the cell fusion, the human-human hybridoma can secrete a human monoclonal antibody capable of recognizing a differentiation antigen of human lymphocytes or exhibiting an immunosuppressive effect against human lymphocytes. The human-human hybridoma which produces the monoclonal antibody of the invention is the 223.10.33 strain deposited at Fermentation Research Institute, Japan, with a deposit No. FERM BP-2384, which has been obtained by the cell fusion

4

between the SA-1 strain, the transformed human B blast cells as the transformed human cells, and the EBV-SU strain, the transformed human antibody-producing B cells as the human antibody-producing cells. The human monoclonal antibody here obtained is novel in respect of the capacity of recognizing a differentiation antigen against human lymphocytes or the immunosuppressive effect against human lymphocytes and is useful in the therapeutic and diagnostic purposes, in particular, as an immunosuppressive agent without the trouble of any side effect on human body.

The medicament form in which the inventive human monoclonal antibody is used as an immunosuppressive agent is not particularly limitative including pills, tablets, granules, powders, capsules, syrups, ointments, liniments, injections and the like. It is of course that each medicament form is prepare dby compounding the monoclonal antibody as the effective ingredient with any pharmacologically acceptable additives conventionally used in medicament preparations.

As to the dose of the medicament to be administrated, it is usual that a sufficient effect as desired can be obtained with 1 mg to 5 g of the administrated medicament calculated as the monoclonal antibody per day per adult.

The monoclonal antibody of the invention has no reactivity with normal T and B cells of human peripheral blood but is reactive with the lymphocytes stimulated with various kinds of mitogens and interleukin-2 (IL-2). Therefore, the monoclonal antibody may provide a means to recognize appearance of a differentiation antigen by the activation of the lymphocytes and is useful in the diagnostic purpose of certain diseases. Namely, a differentiation antigen by the activation of human lymphocytes appears to recognize and eliminate the foreign body by the activity of the immune system when a human body is infected with bacteria or virus or when a human body is suffering a suppurative disease. Since the monoclonal antibody of the invention is capable of recognizing such a human lymphocyte activated and exhibiting a differentiation antigen, the monoclonal antibody can be effectively utilized in an in vivo or in vitro diagnostic purpose of a patient of infectious diseases or after a disease or operation by bringing the biological sample into contact with the monoclonal antibody to form a complex with the lymphocytes and detecting or determining the complex-forming lymphocytes.

As is understood from the above given description, the monoclonal antibody of the invention capable of recognizing a differentiated antigen of human lymphocytes is absolutely free from the danger of any side effects when it is administrated to a human body since it is a product of a human-human hybridoma and not a foreign protein against the human body. Accordingly, application of the monoclonal antibody is not limited to an effective ingredient in an immunosuppressive agent but it can be effectively used widely in the therapeutic and diagnostic fields.

As to the application as an immunosuppressive agent, the monoclonal antibody of the invention as the principal ingredient of an immunosuppressive agent can be used satisfactorily as a rejection-preventing agent in an operation of organ transplantation or as a therapeutic medicament of certain immuno-abnormalities such as autoimmune diseases because, different from conventional immunosuppressive agents such as OKT3 and the like derived from mice, it is derived from a human-human hybridoma and absolutely free from the disadvantage of side effects when it is administrated to a human body.

The monoclonal antibody of the invention can recognize an antigen having a molecular weight in the range of 100 to 105 kDa, 120 to 130 kDa and 270 to 280 kDa for which no detecting means was hitherto known so that the inventive monoclonal antibody may provide a possibility of detecting an antigen which cannot be detected in the prior art.

In the following, examples are given to illustrate the invention in more detail but not to limit the scope of the present invention an any way.

Preparation Example. Preparation of a human-human hybridoma 223.10.33 strain

(I) Preparation of antibody-producing B cells

Peripheral blood was taken from pregnant volunteer (SU: HLA-A2, -: Bw 46.22: Cw 7, -: DR 4,8) and the lymphocytes were collected therefrom by the Conray-Ficoll gradient method. The B cells were obtained by separating the T and B cells by the rosette formation method using SRBC treated with neuraminidase.

(II) Sensitization of B cells with Epstein-Barr virus (EBV)

An EBV strain was prepared in a culture of EBV-transformed marmoset cells, B95-8 strain, growing in a RPMI 1640 culture medium with addition of 10% of fetal calf serum (FCS) and the supernatant containing the viruses was twice filtered. The virus strain was added to 100 $\mu$l of a suspension containing the B cells in a density of $5 \times 10^4$ cells/100 $\mu$l and incubated at 37 °C for 1 hour. Thereafter, a 800 $\mu$l fresh portion of the same 10% FCS-added RPMI 1640 culture medium was added thereto. The incubation was continued by adding the same volume of the fresh culture medium in every 3 to 4 days. Transformed B cells, referred to as the EBV-SU hereinbelow, could be detected about 2 weeks after sensitization.

(III) Treatment of parent cells, EBV-transformed B lymphoblast cells (SA-1), with emetine and actinomycin D

Homozygous SA-1 cells (HLA-A24, B7, C-, DR 1, Dw 1) were cultured in a 10% FCS-added RPMI 1640 culture medium. The cells harvested at the growing phase were washed by centrifugation at 1000 rpm three times each for 10 minutes with the RPMI 1640 medium and conditioned in a density of $1 \times 10^4$ cells/ml followed by a treatment with an emetine hydrochloride solution of $5 \times 10^{-5}$ M concentration and 0.1 $\mu$g/ml of actinomycin D at 37 °C for 2 hours. The above mentioned concentrations of emetine and actinomycin D were sufficient to completely suppress division and proliferation of the SA-1 cells. free emetine and actinomycin D were completely removed from the cells by washing three times with fresh portions of the RPMI 1640 medium to give proliferation-inhibited SA-1 cells.

(IV) Cell fusion and growth conditions for hybrid

The EBV-SU cells collected and washed three times with the RPMI 1640 medium were suspended in the RPMI 1640 medium and mixed with the proliferation-inhibited SA-1 cells in a proportion of 10: 1.

The above prepared cell mixture was admixed with 0.25 ml of a 15% DMSO-42.5% polyethylene glycol (PEG1000) solution taking 1 minute under continuous swilling and then with a 9 ml fresh portion of the RPMI 1640 medium warmed at 37 °C at a rate of 1 ml/minute.

The cells in suspension were consolidated by centrifugation at 1000 rpm for 10 minutes and then suspended in the 10% FCS-added RPMI 1640 medium. The suspension was distributed to the wells of microtiter plates while the density of the cells in the suspension was adjusted such that $5 \times 10^4$ cells were distributed on each well. An additional 0.1 ml fresh portion of the culture medium was added to each well in every 2 to 3 days.

(V) Detection of HLA antigen and selection of fused cells

The surface HLA-DR antigen of the hybridoma was detected by the microlymphocytotoxicity test to conduct selection of the fused cells having the 1, 4 and 8 antigens. Two hybridomas having the HLA-DR 1, 4 and 8 antigens were obtained from a million of the EBV-SU cells. Further, the desired hybridoma was obtained by the limiting dilution method.

In the next place, this hybridoma 223 was subjected to cloning in a density of 10 cells/well to give a subclone 223.10.33 strain.

<u>Example 1.</u> Preparation of immunoglobulin M (IgM) by the 223. 10. 33 strain in serum-added culture medium

The cells of the 223. 10. 33 strain obtained in the Preparation Example were subcultured in a 10% FCS-added RPMI 1640 culture medium contained in a flask at 37 °C under an atmosphere of a gaseous mixture of 5% carbon dioxide and 95% air. The thus subcultured cells were dispersed in a 5 liters fresh portion of the same culture medium in a cell density of $2 \times 10^5$ cells/ml and agitation culture was performed for about 6 days in a spinner flask of 5 liters capacity rotated at 20 rpm with a concentration of dissolved oxygen of 4 ppm to give a culture containing about $1 \times 10^6$ cells/ml. The cells were removed from the culture by centrifuging at 4000 rpm for 5 minutes to give 5 liters of the supernatant which was subjected to ultrafiltration using a membrane capable of eliminating particles having a molecular weight smaller than about 300,000 to reduce the volume to 500 ml. Ammonium sulfate was added to the thus concentrated supernatant in a concentration of 50% saturation and the mixture was centrifuged to collect the precipitates as an immunoglobulin (IgM) -containing crude product. This crude product was dissolved in 150 ml of a 10 mM phosphate buffer solution having a pH of 7.4 and containing 0.9% of sodium chloride. The solution was dialyzed against the same buffer solution for about 18 hours to give about 200 ml of a dialyzed solution. A chromatographic column having an inner diameter of 5 cm and a height of 90 cm filled with Sephacryl S-300 was loaded with 100 ml of the thus obtained dialyzed solution and elution was performed with the same buffer solution. Determination of IgM in the eluate fractions was undertaken by the photometric measurement at a wavelength of 280 nm and IgM determination by the ELISA method. The IgM-containing fraction in a volume of about 250 ml was subjected to ultrafiltration to give a concentrated solution in a volume of 6 ml containing an antibody against the 223. 10. 33 strain. This antibody solution contained 1 mg/ml of the IgM and the titer thereof was 256 times by the complement-dependent cellular cytotoxic assay.

Example 2. Preparation of IgM by the 223. 10. 33 strain in GIT culture medium

The culture medium used here was a commercially available GIT culture medium admixed with a cell growth factor in an animal serum in place of the fetal calf serum.

The cells of the 223. 10. 33 strain obtained in the Preparation Example were subcultured in the above mentioned culture medium contained in a flask at 37 °C under an atmosphere of a gaseous mixture of 5% carbon dioxide and 95% air. The thus subcultured cells collected by centrifugation at 1500 rpm for 5 minutes were added to 20 flasks of each 75 cm$^2$ size containing a 30 ml fresh portion of the GIT culture medium and suspended therein in a density fo 4. 6 $\times$ 10$^5$ cells/ml. The suspensions in the 20 flasks were combined to make up a volume of 600 ml and introduced into a spinner flask of 2 liters capacity together with a 1400 ml fresh portion of the same culture medium to give a cell density of 2 $\times$ 10$^5$ cells/ml as dispersed followed by further continued agitation culture at 32 rpm with a concentration of dissolved oxygen of 4 ppm.

When the cell density had reached 1 $\times$ 10$^6$ cells/ml after 6 days of culturing, the cells were removed from the culture by centrifugation at 4000 rpm for 5 minutes to give 2 liters of the supernatant. The supernatant was passed down through a chromatographic column of 0.5 cm inner diameter and 15 cm length filled with Sepharose CL-6B loaded with protein A at a flow rate of 1 ml/minute to have the IgM adsorbed on the filler followed by washing with a 0.02 M phosphate buffer solution having a pH of 4 at a flow rate of 1 ml/minute and then elution of the IgM with a 0.03 M phosphate buffer solution having a pH of 3 at a flow rate of 1 ml/minute. Detection of the IgM in the eluate fractions was undertaken by the photometric measurement at a wavelength of 280 nm and determination of IgM by the ELISA method.

Gel-filtration chromatography by FPLC was undertaken for 200 ml of the thus obtained IgM-containing fraction through a Pharmacia Superose 6 column (a product by Pharmacia Co.) using a 0.01 M phosphate buffer solution having a pH of 6.5 at a flow rate of 0.5 ml/minute as the eluant and the IgM-containing fraction in a volume of 100 ml was subjected to ultrafiltration using a membrane capable of eliminating particles having a molecular weight smaller than about 300,000 to give 10 ml of a concentrated solution containing an antibody against the 223. 10. 33 strain corresponding to 10 mg of the IgM. This antibody was identified to be pure by the result of the SDS electrophoresis. The titer of this antibody solution was 512 times as determined by the complement-dependent cytotoxicity assay.

Example 3. Reactivity of the 223. 10. 33 antibody with mononuclear cells fo peripheral blood: complement-dependent cytotoxicity assay

The peripheral blood samples taken from 18 normal persons were subjected to the Conray-Ficoll method to isolate the mononuclear cells which were further separated into the T cells and B cells by the nylon column method and sheep red cells rosette formation method. Experiments were conducted by using these T and B cells as well as the peripheral blood mononuclear cells stimulated with three kinds of mitogens including PHA, i.e. phytohemagglutinin, PWM, i.e. pokeweed mitogen, and ConA, i.e. concanavalin A, and IL-2, i.e. interleukin 2. Thus, the PHA, PWM, ConA and IL-2 were added each to a RPMI 1640 culture medium containing 10% of human serum in a concentration of 0.1%, 1%, 10 $\mu$g/ml and 1 u, respectively, and cultured for 5 days to effect stimulation. A 1 $\mu$l portion of the antibody solution prepared in Example 1 was added to a Terasaki plate and the above described target cells wwere poured portionwise to each of the wells to give 2000 cells/GVB$^{++}$ with a gelatin-Veronal ¯buffer solution and the reaction was effected at 37 °C for 1 hour. Thereafter, 5 $\mu$l of a twice-diluted rabbit complement (a product by Peritus Co.) were added thereto to effect the reaction for 2 hours at room temperature followed by eosin stain and formalin fixation to microscopically examine life or death of the cells. The results of the reactivity as given by the ratio of the number of positive cells to the total number of the examined cells were as follows.

| Peripheral blood T cells: | 0/18 |
| Peripheral blood B cells: | 0/18 |
| PHA-stimulated peripheral blood mononuclear cells: | 5/5 |
| PWM-stimulated peripheral blood mononuclear cells: | 5/5 |
| ConA-stimulated peripheral blood mononuclear cells: | 3/5 |
| IL-2-stimulated peripheral blood mononuclear cells: | 5/5 |

The above given results indicate that the 223.10.33 antibody was reactive with the peripheral blood mononuclear cells stimulated with the mitogens or IL-2 but non-reactive with the unstimulated peripheral blood T cells and B cells.

Example 4. Reactivity of the monoclonal antibody with activated human T cells

The peripheral blood smaples taken from 3 normal persons were subjected to the Conray-Ficoll method to isolate the mononuclear cells from which the T cells were separated by the sheep red cells rosette formation method. These cells were suspended in a RPMI 1640 culture medium with admixture of 20% of human serum and containing 10 u or 1 u of IL-2 in a cell density of $1 \times 10^5$ cells/ml and cultured at 37 °C under an atmosphere of a gaseous mixture of 5% carbon dioxide and 95% air with periodical replacement of a half volume of the culture medium once in every three days. The thus cultured cells were collected from each culture medium after 1, 2, 3, 5 and 7 days fo culturing as well as before the start of culturing and microscopically examined for life or death of the cells by the cytotoxicity assay in the same manner as in Example 3 to give the results shown in Table 1 below by the percentage of the injured T cells on an average for the 3 persons. As is shown in the table, the reactivity of the 223.10.33 antibody was apparent after 5 days of culturing and later on.

Table 1

| IL-2 stimulant concentration | Days of culturing | | | | | |
| --- | --- | --- | --- | --- | --- | --- |
| | 0 | 1 | 2 | 3 | 5 | 7 |
| 10 u | 0 | 0 | 10 | 20 | 80 | 60 |
| 1 u | 0 | 0 | 20 | 10 | 50 | 30 |

Example 5. Reactivity of the monoclonal antibody with transformed human B cells

The peripheral blood samples taken from 3 normal persons were subjected to the Conray-Ficoll method to isolate the mononuclear cells from which the B cells were separated by the sheep red cells rosette formation method. These B cells were transformed with the Epstein-Barr virus to give EBV-B cells according to the procedure described in Nature, volume 269, pages 420-422 (1977). The reactivity of the antibody with these cells was examined in the same manner as in Example 3 by continuing the culturing up to 34 days to give the results that the antibody was non-reactive with the cells until 9 days of culturing but reactive after 12 or 14 days of culturing to give the percentages of the injured cells shown in Table 2 below.

Table 2

| Days of culturing | 9 | 12 | 14 | 16 | 19 | 21 | 24 | 34 |
| --- | --- | --- | --- | --- | --- | --- | --- | --- |
| % injured cells, average | 0 | 20 | 50 | 50 | 70 | 50 | 70 | 80 |

Example 6. Influence on the antibody production of mononuclear cells

Mononuclear cells were isolated from normal human peripheral blood by the Conray-Ficoll method. The cells were suspended in a RPMI 1640 culture medium containing 1% of PWM and admixed with 10% of

8

human serum in a density of $5 \times 10^5$ cells/ml and cultured at 37 °C using a 96-well U-shaped microplate under an atmosphere of a gaseous mixture of 5% carbon dioxide and 95% air. At the moments of 1, 3, 6, 12, 24, 48, 72, 96 and 120 hours from the start of culturing, a 20 μl portion of an antibody solution dissolved in the RPMI 1640 culture medium was added to each well. The antibody solution was a 5 mg/ml solution of the 223.10.33 antibody or a 5 mg/ml solution of the standard human IgM supplied by Kappel Co. After a week of continued culturing, the cells were recovered from the culture by centrifugation and subjected to detection of the IgC-reactive and IgM-reactive plaque-forming cells (PFC) by the reverse plaque method descirbed in "Manual of Immunological Experiments", C, pages 2003-2004 to give the results shown in Table 3, in which the numbers of the IgG-reactive and IgM-reactive PFCs (IgG-PFC and IgM-PFC) are given per $10^4$ of the total cells. These results indicate that the 223.10.33 antibody clearly had an activity of inhibiting production of IgG and IgM throughout the period from immediately after stimulation with PWM to the stage of differentiation and proliferation of the cells while the commercially available human IgM had no such an activity.

Table 3

| PWM | Culturing time up to addition of antibody, hours | IgG-PFC/$10^4$ | | IgM-PFC/$10^4$ | |
|---|---|---|---|---|---|
| | | 223.10.33 antibody added | Human IgM added | 223.10.33 antibody added | Human IgM added |
| No | | 0 | 0 | 0 | 0 |
| Yes | | 171 | 171 | 266 | 266 |
| Yes | 0 | 0 | 134 | 0 | 230 |
| Yes | 1 | 0 | 81 | 0 | 177 |
| Yes | 3 | 0 | 281 | 0 | 499 |
| Yes | 6 | 0 | 135 | 0 | 310 |
| Yes | 12 | 0 | 132 | 0 | 243 |
| Yes | 24 | 0 | 117 | 0 | 227 |
| Yes | 48 | 0 | 215 | 18 | 358 |
| Yes | 72 | 5 | 141 | 2 | 258 |
| Yes | 96 | 0 | 200 | 5 | 276 |
| Yes | 120 | 0 | 172 | 0 | 327 |

Example 7. Assay for the molecular weight of recognized antigens

The Western-blot technique was applied to the assay of the molecular weight of the recognized antigens. Thus, B-85 cells, i.e. B lymphoblast-like cell line transformed with EBV, and BT-1 cells, i.e. Burkitt lymphoma, having particularly high reactivity with the 223.10.33 antibody were solubilized with 0.5% NP-40 and nuclei were removed by centrifugation to give a fraction containing the cell membranes. This fraction was subjected to electrophoresis with an SDS polyacrylamide gel. Thereafter, transcription was undertaken from the gel to a nitrocellulose filter followed by the reaction with the 223.10.33 antibody to detect the recognized antigens using a goat IgM labelled with peroxidase (POD) (a product by Kappel Co.). As a result, three developed bands were detected respectively corresponding to the molecular weights of 100 to 105 kDa, 120 to 130 kDa and 270 to 280 kDa.

Example 8. B cell marker detectable with the monoclonal antibody

Reactivity of the 223.10.33 antibody was examined using strains of B cells at different stages of differentiation as the target cells by the indirect fluorescent antibody technique described in Handbook in Clinical Immunology, page 1510 (1984).

The CD antibodies [see Clinical Immunology, volume 18, Summerly Special Issue, page 149 (1986)] used here were B 2 (CD 21, coal tar clone), Leu-14 (CD 22, a product by Fujisawa Yakuhin Co.) and H 107 (CD 23, a product by Nitiray Co.). The results are shown in Table 4, from which it is confirmed that the 223.10.33 abtibody is closely correlated with the CD 23 antibody.

Table 4

| Differentiation stages and cell strains / Monoclonal antibody | Pre-B cells | | | B lymphocytes | | | | plasma cells | |
|---|---|---|---|---|---|---|---|---|---|
| | | | | immature/resting | | mature/activated | | | |
| | Ramos | Daudi | BALL-1 | RAJI MGAR SPACHEO MZ070782 PF97387 | AKIBA RSH OLL HID | BM16 Sa-IV AMALA EK/OH | KT-12 COX SP0010 RML | B-85 BT-1 MANN | RPMI8226 |
| B2 (CD21) EBV recepter C3D recepter | - | + | + | + | + | +! | - | - | - |
| Leu-14 (CD22) | + | + | - | + | + | + | + | - | - |
| H107 (CD23) Fc recepter | - | - | - | + | + | + | + | + | + |
| 223.10.33 | - | - | - | + | + | + | + | + | - |

Note: +: positive, -: negative, !: false positive

10

## Claims

1. A monoclonal antibody derived from a human-human hybridoma which has been deposited under the deposit number FERM BP-2384 and capable of recognizing a differentiation antigen of human lymphocytes or capable of exhibiting an immunosuppressive activity against human lymphocytes.

2. A monoclonal antibody as claimed in claim 1 wherein the human-human hybridoma is prepared by subjecting a transformed human cell to a proliferation-inhibiting treatment and then to cell fusion with a human antibody-producing cell.

3. An antibody derived from a human-human hybridoma which has been deposited under the deposit number FERM BP-2384 for use in a method for immunosuppression comprising administrating it to a human body as an immunosuppressive agent.

4. An antibody as claimed in claim 3 wherein the human-human hybridoma is prepared by subjecting a transformed human cell to a proliferation-inhibiting treatment and then to cell fusion with a human antibody-producing cell.

5. A method for detecting or determining a differentiation antigen in a biological sample which comprises contacting the biological sample with a monoclonal antibody derived from a human-human hybridoma which has been deposited under the deposit number FERM BP-2384, the antibody being capable of recognizing the differentiation antigen of human lymphocytes, so as to form a complex of the monoclonal antibody and any lymphocytes in the biological sample having said differentiation antigen and detecting or determining the complex.

6. The use of a monoclonal antibody derived from a human-human hybridoma which has been deposited under the deposit number FERM BP-2384 in preparing a medicament for immunosuppressive purposes.

7. The use of a monoclonal antibody derived from a human-human hybridoma which has been deposited under the deposit number FERM BP-2384 in a method of diagnosis not being one carried out on the human or animal body.

8. An immunosuppressive agent comprising a monoclonal antibody as defined in claim 1 or claim 2 as an active ingredient.

## Patentansprüche

1. Monoklonale Antikörper, die von einem Human-Human-Hybridom abgeleitet sind, das unter der Hinterlegungsnummer FERM BP-2384 hinterlegt ist, und die befähigt sind, ein Differenzierungs-Antigen menschlicher Lymphocyten zu erkennen oder eine immunsuppressive Wirksamkeit gegenüber menschlichen Lymphocyten auszuüben.

2. Monoklonale Antikörper nach Anspruch 1, wobei das HumanHuman-Hybridom durch die Vermehrung inhibierende Behandlung einer transformierten menschlichen Zelle und anschließende Zellfusion mit einer menschlichen, Antikörper produzierenden Zelle hergestellt ist.

3. Antikörper, die von dem unter der Hinterlegungsnummer FERM BP-2384 hinterlegten Human-Human-Hybridom abgeleitet sind, zur Verwendung bei einem Verfahren zur Immunsuppression, bei dem die Antikörper als immunsuppressives Mittel einem Menschen verabreicht werden.

4. Antikörper nach Anspruch 3, wobei das Human-Human-Hybridom durch die Vermehrung inhibierende Behandlung einer transformierten menschlichen Zelle und anschließende Zellfusion mit einer menschlichen, Antikörper produzierenden Zelle hergestellt ist.

5. Verfahren zur Erfassung oder Bestimmung von Differenzierungs-Antigenen in biologischen Proben durch Inkontaktbringen der biologischen Probe mit einem monoklonalen Antikörper, der von einem Human-Human-Hybridom abgeleitet ist, das unter der Hinterlegungsnummer FERM BP-2384 hinterlegt ist, und der befähigt ist, ein Differenzierungs-Antigen menschlicher Lymphocyten zu erkennen, so daS ein Komplex des monoklonalen Antikörpers mit Lymphocyten in der biologischen Probe gebildet wird, die dieses Differenzierungs-Antigen aufweisen, und Erfassung oder Bestimmung des Komplexes.

6. Verwendung von einem Human-Human-Hybridom, das unter der Hinterlegungsnummer FERM BP-2384 hinterlegt ist, abgeleiteter monoklonaler Antikörper zur Herstellung von Arzneimitteln zur Immunsuppression.

7. Verwendung von monoklonalen Antikörpern, die von einem Human-Human-Hybridom abgeleitet sind, das unter der Hinterlegungsnummer FERM BP-2384 hinterlegt ist, in Diagnoseverfahren, die nicht am menschlichen oder tierischen Körper durchgeführt werden.

8. Immunsuppressives Mittel, das als Wirkstoff einen monoklonalen Antikörper nach Anspruch 1 oder 2 enthält.

**Revendications**

1. Anticorps monoclonal dérivé d'un hybridome humain-humain, qui a été déposé sous le numéro de dépôt FERM BP-2384 et capable de reconnaître un antigène de différenciation des lymphocytes humains ou capable de montrer une activité immunosuppressive contre les lymphocytes humains.

2. Anticorps monoclonal selon la revendication 1, dans lequel l'hybridome humain-humain est préparé en soumettant une cellule humaine transformée à un traitement inhibant la prolifération et ensuite à une fusion cellulaire avec une cellule produisant un anticorps humain.

3. Anticorps dérivé d'un hybridome humain-humain, qui a été déposé sous le numéro de dépôt FERM BP-2384, pour utilisation dans une méthode pour immunosuppression, comprenant son administration à un corps humain, en tant qu'agent immunosuppresseur.

4. Anticorps selon la revendication 3, dans lequel l'hybridome humain-humain est préparé en soumettant une cellule humaine transformée à un traitement d'inhibition de la prolifération et ensuite à une fusion cellulaire avec une cellule produisant des anticorps humains.

5. Méthode pour détecter ou déterminer un antigène de différenciation dans un échantillon biologique qui comprend la mise en contact de l'échantillon biologique avec un anticorps monoclonal dérivé d'un hybridome humain-humain, qui a été déposé sous le numéro de dépôt FERM BP-2384, l'anticorps étant capable de reconnaître l'antigène de différenciation de lymphocytes humains, de façon à former un complexe de l'anticorps et de tout lymphocyte dans l'échantillon biologique ayant ledit antigène de différenciation et la détection ou la détermination du complexe.

6. Utilisation d'un anticorps monoclonal dérivé d'un hybridome humain-humain, qui a été déposé sous le numéro de dépôt FERM BP-2384 dans la préparation d'un médicament pour des buts immunosuppresseurs.

7. Utilisation d'un anticorps monoclonal dérivé d'un hybridome humain-humain qui a été déposé sous le numéro de dépôt FERM BP-2384 dans une méthode de diagnostic n'étant pas mise en oeuvre sur le corps humain ou animal.

8. Agent immunosuppresseur, comprenant un anticorps monoclonal selon la revendication 1 ou 2, en tant qu'ingrédient actif.